# FASCICULE DE BREVET EUROPEEN

(11) **EP 3 302 613 B1**
(45) Date de publication et mention de la délivrance du brevet: **25.12.2019**
(21) Numéro de dépôt: 16736620.2
(22) Date de dépôt: 02.06.2016
(51) Int. Cl.: A61M 1/28

(54) **SYSTEME DE TRAITEMENT DE DIALYSE PERITONEALE**
SYSTEM ZUR PERITONEALDIALYSEBEHANDLUNG
PERITONEAL DIALYSIS TREATMENT SYSTEM

(30) Priorité: 03.06.2015 EP 15170614
(43) Date de publication de la demande: 11.04.2018
(73) Titulaire: Debiotech S.A., 1004 Lausanne (CH)
(72) Inventeur: NEFTEL, Frédéric, 1005 Lausanne (CH)
(74) Mandataire: Weihs, Bruno Konrad
(86) Numéro de dépôt international: PCT/IB2016/053241
(87) Numéro de publication internationale: WO 2016/193930

(56) Documents cités:
- WO-A1-2016/095026
- US-B1- 6 228 047

## Description

### Domaine de l'invention

La présente invention concerne un système de dialyse adapté pour effectuer un retrait d'une fraction volumique de dialysat de la cavité péritonéale d'un patient en traitement, durant une phase de stase, de manière à optimiser le traitement (par exemple l'ultrafiltration), la sécurité du patient, le confort du patient et/ou le fonctionnement du système.

### Etat de la technique

La dialyse péritonéale est un moyen thérapeutique permettant de purifier le sang. Elle permet, à un patient souffrant d'une insuffisance rénale, d'éliminer les impuretés telles que l'urée et l'excès d'eau de l'organisme qui auraient été habituellement éliminés par des reins fonctionnant normalement. Ce moyen thérapeutique utilise le péritoine du patient. La membrane péritonéale dispose d'une très grande surface et elle comporte de très nombreux vaisseaux sanguins. Elle joue ainsi le rôle de filtre naturel entre le sang et tout liquide (le dialysat) éventuellement présent dans la cavité péritonéale. De nombreux brevets et demandes de brevet divulguent des systèmes permettant d'effectuer des traitements par dialyse péritonéale, tels que les documents portant les numéros suivants EP 1 648 536 A2, EP 0 471 000 B1, EP 1 195 171 B1, EP 1 648 536 B1, US6228047 B1 et WO2016/095026 A1, dont le dernier est compris dans l'état de la technique selon l'article 54(3) CBE.

Un traitement par dialyse péritonéale est relativement simple et comprend au moins un cycle de trois phases distinctes :
- le « Fill » (également appelé phase de remplissage): le système injecte du dialysat dans la cavité péritonéale du patient ;
- le « Dwell » (également appelé phase de stase): le système laisse durant un temps déterminé le dialysat dans la cavité péritonéale ;
- le « Drain » (également appelé phase de drainage): le système retire le dialysat présent dans la cavité péritonéale.

Dans le présent document, une phase peut être un Fill, un Dwell ou un Drain (chaque phase pouvant être totale ou partielle), un cycle complet comprend un Fill, un Dwell et un Drain. Le premier cycle peut ne comprendre qu'une phase de drainage lorsque que le dernier cycle du traitement précédent ne comprenait qu'une phase de remplissage. Un traitement peut comprendre plusieurs cycles, autrement dit, les phases peuvent être répétées plusieurs fois durant un même traitement.

Les systèmes généralement appelés dispositifs d'APD (Automated Peritoneal Dialysis) sont adaptés pour effectuer plusieurs enchainements de phases de Fill, de Dwell et de Drain qui se succèdent, autrement dit plusieurs cycles se succédant durant un même traitement. Ce type de système effectue ainsi un traitement pendant plusieurs heures. Aussi, les dispositifs d'APD sont particulièrement adaptés pour une utilisation durant la nuit et/ou chez le patient.

Durant le Dwell, le dialysat injecté dans la cavité péritonéale du patient évolue dans le temps, sa composition est modifiée du fait de l'échange de composants à travers la membrane péritonéale et son volume varie en fonction de l'efficacité de l'ultrafiltration, c'est-à-dire de la quantité de liquide retiré du patient (par exemple l'eau) et/ou réabsorbé par ledit patient. En conséquence la pression intrapéritonéale varie durant le traitement. Cette pression est liée, notamment, à la quantité de liquide présente dans la cavité péritonéale. Plus le volume de dialysat injecté durant la phase de Fill est important, plus la pression initiale sera importante, de même plus l'ultrafiltration sera efficace durant la phase de stase, et plus la pression augmentera au cours de cette phase. Toutefois, au-delà d'un certain seuil, plus la pression intrapéritonéale augmente moins l'ultrafiltration pourra s'effectuer. Diverses publications traitent de ce sujet et, notamment, celle du Dr. Michel Fischbach "Use of intraperitoneal pressure, ultrafiltration and purification dwell times for individual peritoneal dialysis prescription in children" (Clinical nephrology 1996 jul;46(1): 14-6).

Au niveau clinique, il est souhaitable d'utiliser un grand volume de dialysat durant une longue durée de Dwell pour améliorer l'ultrafiltration, mais la pression intrapéritonéale sera fortement impactée et au-delà d'un certain seuil de pression, l'ultrafiltration ne sera pas efficace. De plus, plus la pression intrapéritonéale est élevée plus une sensation d'inconfort est ressentie par le patient et une pression intrapéritonéale trop élevé peut diminuer la capacité vitale du patient et/ou affecter sa santé en réduisant, par exemple, sensiblement le volume de réserve pulmonaire. Ainsi, la pression intrapéritonéale permet d'apprécier la tolérance du volume intrapéritonéal et également à optimiser l'ultrafiltration. En conséquence, le volume de dialysat injecté durant la phase de Fill et/ou la pression intrapéritonéale (qui varie durant la phase de stase) est/sont des données importantes. Cette pression peut varier également lorsque le patient bouge, respire ou se lève. Par exemple, la pression augmente lorsque le patient inspire et diminue lorsque le patient expire. De la même façon, la pression variera en fonction des mouvements du patient par exemple quand le patient bouge, se tourne dans son lit, se lève, se met en position assise, tousse, éternue,...

Certains dispositifs de l'état de l'art limitent la quantité de dialysat injecté durant la phase de Fill, de manière à ce que la pression intrapéritonéale (durant la phase de stase) reste inférieure à un seuil qui, au-delà, engendrerait une sensation d'inconfort pour le patient. Cela suggère que le système est capable de prédire la variation de la pression intrapéritonéale durant la phase de stase. Cette prédiction est complexe et souvent inefficace, car chaque patient est différent et la pression intrapéritonéale peut également varier en fonction de l'état de santé du patient ou de sa volémie.

D'autres systèmes de l'art mesurent en permanence la pression intrapéritonéale de manière à maintenir cette pression dans un intervalle donné. Ainsi, durant la phase de Fill, le système injecte le dialysat dans la cavité jusqu'à ce que la pression intrapéritonéale atteigne une certaine valeur. Durant la phase de stase, le système surveille en continu cette pression, retire du dialysat lorsque la pression intrapéritonéale dépasse un seuil plafond et réinjecte du dialysat lorsque cette pression atteint un seuil plancher. Ces systèmes effectuent ainsi un grand nombre d'opérations durant la phase de stase, faisant varier sensiblement le volume de dialysat dans la cavité tout au long de la phase de stase, engendrant ainsi une certaine incertitude sur la réelle efficacité d'ultrafiltration. D'autant plus que lorsque que le patient bouge, par exemple durant son sommeil, le système détectera une variation de la pression qui ne sera pas due à l'ultrafiltration, le système réinjectera ou retirera ainsi du dialysat, puis lorsque le patient reviendra dans sa position initiale le système effectuera l'opération inverse.

### Description générale de l'invention

L'invention présentée dans ce document est définie par les revendications ci-jointes et apporte plus d'intelligence à un système de dialyse de manière à améliorer l'efficacité du traitement, le confort et la sécurité du patient et/ou le fonctionnement du système. L'invention est particulièrement intéressante pour les APD qui sont utilisés la nuit ou chez le patient car dans ces deux cas, il n'est pas nécessaire d'avoir un médecin près du système pour initier le retrait d'un volume de dialysat pendant la phase de stase.

Le système de l'invention est adapté de manière à permettre le retrait d'un volume donné de dialysat présent dans la cavité péritonéale du patient durant la phase de stase. Un des objectifs de l'invention est de limiter autant que possible le retrait de dialysat lorsque le patient bouge ou plus généralement lorsque la pression intrapéritonéale varie sans que cela soit dû à une augmentation résultant de l'ultrafiltration. Ainsi le système ne peut commander ce retrait.

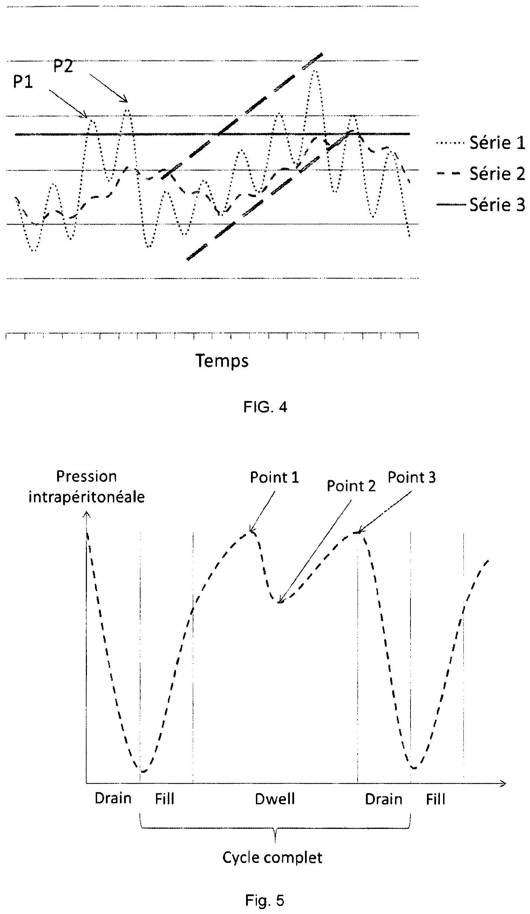

La figure 5 expose un profil de pression durant une partie d'un traitement de dialyse. Les données de pression sont liées à la pression intrapéritonéale du patient. On peut remarquer à travers ce graphique que la pression intrapéritonéale évolue dans le temps durant la phase de stase. Cette évolution de pression peut être substantiellement liée à l'ultrafiltration. Durant les phases de remplissage et de drainage, la variation de pression est substantiellement dû au déplacement du dialysat vers ou en dehors de la cavité péritonéale. Le point 1 peut être le moment où le retrait partiel durant la phase de stase est déclenché, le point 2 peut être le moment où le retrait partiel durant la phase de stase est arrêté et le point 3 peut être le moment où le retrait du dialysat est déclenché du fait du commencement de la phase de drainage. Dans cet exemple, on peut estimer que la quantité retirée de dialysat durant la phase de stase correspond substantiellement à la quantité d'ultrafiltrat (produit entre le début de la phase de stase et le point 1 ou 2) car le niveau de pression au point 2 est substantiellement au même niveau de pression qu'en fin de phase de remplissage.

### Liste des figures

L'invention sera mieux comprise ci-après au moyen de quelques exemples illustrés. Il va de soi que l'invention ne se limite pas à ces modes de réalisation.
**Figure 1** schématise un mode de réalisation d'un système de dialyse
**Figure 2** schématise le positionnement des éléments du système par rapport au circuit du dialysat
**Figure 3** expose les fenêtres de temps possible
**Figure 4 et 5** illustrent des profils des pressions.

### Références numériques utilisées dans les figures

- 100: Système
- 101: Patient
- 102: Cycler
- 103: Cassette
- 104: Conteneur
- 105: Conteneur
- 106: Contrôleur (par exemple Processeur)
- 107: Actuateur de pompe
- 108: Capteur de pression
- 109, 110, 111: Autres éléments connectés aux processeurs (écran, capteur, clavier, bouton,...)
- 300: Système
- 301: Patient, Cavité péritonéale
- 302: Conteneur de dialysat utilisable
- 303: Conteneur de dialysat utilisé
- 304, 305, 306, 312: Elément d'occlusion (valve automatique, clamp,...)
- 307: Pompe
- 308, 309, 310: Capteur de pression

### Description détaillée de l'invention

Dans le présent document, la description détaillée de l'invention comporte des modes de réalisation de dispositifs, de systèmes et de méthodes présentés à titre d'illustration. Il est bien entendu que d'autres modes de réalisation sont envisageables et peuvent être apportées. La description détaillée qui suit, par conséquent, ne doit pas être prise dans un sens limitatif.

Sauf indication contraires, les termes scientifiques et techniques utilisés dans le présent document ont des significations couramment utilisés par l'homme du métier. Les définitions apportées dans ce document sont mentionnées afin de faciliter la compréhension des termes fréquemment utilisés et ne sont pas destinées à limiter la portée de l'invention.

Les indications de direction utilisées dans la description et les revendications, telles que "haut", "bas", "gauche", "droite", "supérieur", "inférieur", et autres directions ou orientations sont mentionnées afin d'apporter plus de clarté en référence aux figures. Ces indications ne sont pas destinées à limiter la portée de l'invention.

Les verbes « avoir », « comprendre », « inclure » ou équivalent sont utilisés dans le présent document dans un sens large et signifie de façon générale « inclut, mais sans s'y limiter.

Le terme "ou" est généralement employé dans un sens large comprenant "et/ou" à moins que le contexte indique clairement le contraire.

### Systèmes de dialyse adaptés pour l'invention :

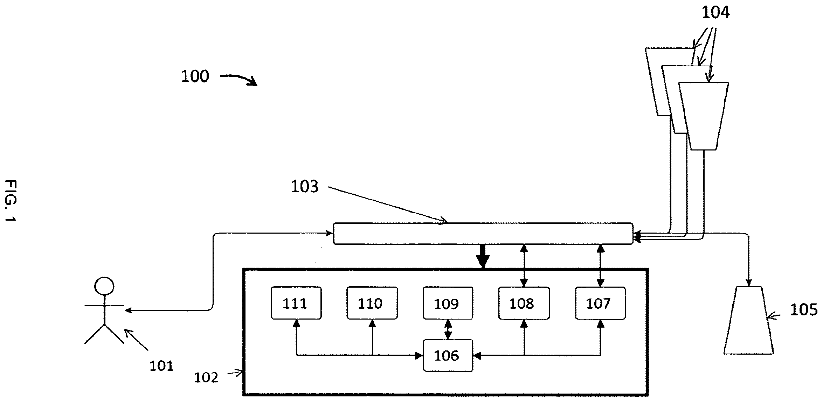

Selon un mode de réalisation divulgué par la figure 1, le système (100) comprend un cycler (102) conçu pour recevoir une cassette jetable (103) qui est en communication fluidique avec la cavité péritonéale du patient et un réservoir adapté pour contenir du dialysat (104, 105). Par exemple, le système peut comprendre un ou plusieurs sacs de dialysat frais (104) et/ou un ou plusieurs sacs de dialysat usagé (105) destiné à recevoir du dialysat utilisé par le traitement. Le cycler peut comprendre des éléments d'actionnement tels que des actuateurs de pompe (107) ou de valves. Ces éléments d'actionnement peuvent être adaptés pour coopérer de façon opérationnelle (par exemple couplage temporaire mécanique) avec la casette jetable (103), par exemple si la cassette comprend des valves ou un système de pompage. Une description complète est décrite dans les demandes internationales suivantes WO 99/51287 A1, WO 2005/009511 A2 et WO 2005/009512 A1. Par jetable, il faut comprendre que la cassette peut être utilisée qu'un certain nombre de fois définit par exemple un unique traitement par rapport au cycler qui est réutilisable plusieurs fois par exemple pour plusieurs traitements. Ainsi un cycler peut être utilisé successives avec plusieurs cassettes jetables.

Le système comprend un capteur adapté pour déterminer, mesurer ou estimer une pression associée à la pression intrapéritonéale. Ce capteur peut être un capteur de pression en communication de pression avec la cavité péritonéale. Ce capteur peut être agencé le plus proche possible de la cavité, sur la ligne reliant le patient à la cassette ou être adapté pour coopérer de façon opérationnelle (par exemple par couplage mécanique et temporaire) avec la cassette. Le mode de réalisation exposé par la figure 1 présente un capteur de pression (108) agencé dans le cycler et coopérant avec la cassette. La cassette (103) comprend alors une zone spécifique avec une membrane de manière à transférer la pression du dialysat au capteur de pression (108). Une description complète est décrite dans les demandes internationales suivantes WO 2007/085993 A1 et WO 2014/020501 A1. Préférentiellement, cette zone spécifique est en communication de pression et/ou en communication fluidique avec la cavité péritonéale. Ainsi, le capteur de pression peut mesurer la pression d'un fluide en communication fluidique ou de pression avec le fluide présent dans la cavité péritonéale du patient.

Idéalement, le capteur de pression mesure la pression du fluide présent dans la cavité intrapéritonéale, toutefois en fonction de la distance entre la cavité et le capteur et/ou en fonction des matériaux utilisés (tube plus ou moins compliant, par exemple) entre ces deux éléments, il se peut que la pression mesurée par le capteur ne soit pas exactement égal à la pression intrapéritonéale. Ceci est particulièrement vrai quand la pompe est actionnée (lors de la phase de remplissage ou de drainage). En phase de stase, la pression mesurée peut représenter une valeur reflétant substantiellement la pression intra-péritonéale. Toutefois, la mesure parfaite n'est pas indispensable car le système peut se contenter de connaitre uniquement l'évolution de la pression. En effet, même si la pression mesurée n'est pas exactement celle de la pression intrapéritonéale, l'évolution dans le temps de cette pression est idéalement substantiellement identique ou proportionnelle ou similaire à l'évolution de la pression mesurée.

Ainsi, le système peut simplement mesurer une pression associée à la pression intrapéritonéale. Cette mesure peut être égale à la pression intrapéritonéale mais si ce n'est pas le cas alors le système peut utiliser un modèle mathématique pour estimer une valeur relativement proche de la pression intrapéritonéale ou simplement apprécier l'évolution de la pression.

D'autres éléments peuvent être compris dans le système, tel qu'un capteur de température, un moyen de chauffage du dialysat, une interface graphique, un contrôleur électronique (106) (par exemple un processeur, une mémoire couplée au processeur,...

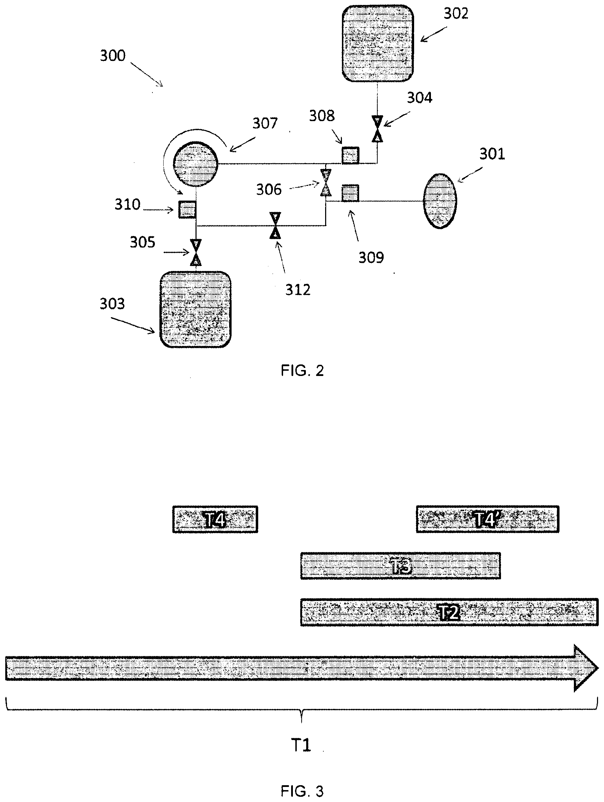

La figure 2 illustre le chemin fluidique d'un mode de réalisation possible. Le système (300) comprend au moins un capteur (308, 309, 310) et un dispositif de distribution de fluide permettant de relier fluidiquement un approvisionnement de dialysat frais (302), un patient (représenté par sa cavité péritonéale (301)) et un réservoir à dialysat usé (303). Le système est conçu pour que le dialysat s'écoule :
- de l'approvisionnement de dialysat frais (302) à la cavité péritonéale (301) du patient,
- de la cavité péritonéale (301) du patient au réservoir à dialysat usé (303).

Une pompe (307) et un ensemble de valves (304, 312, 305, 306) contrôlées par un processeur pour commander automatiquement le déplacement du dialysat ainsi que le chemin de distribution du dialysat. La pompe peut être une pompe péristaltique et peut être à sens unique.

Par exemple, lors d'une phase de Fill, certaines valves (304, 312) sont ouvertes, les autres valves (306, 305) sont fermées et la pompe est actionnée. Les capteurs de pression (308, 309, 310) peuvent surveiller la pression dans le chemin fluidique. Durant la phase de stase, les valves (304, 306, 312, 305) peuvent être fermées. Dans ce mode de réalisation, le capteur de pression (309) reste en communication de pression et fluidique avec la cavité péritonéale (301) du patient. Durant une phase de Drain, la valve 304 est préférentiellement fermée, la valve 312 est fermée, les valves 305 et 306 sont ouvertes et la pompe (307) est actionnée. Les capteurs de pression (308, 310,309) peuvent surveiller la pression dans le chemin fluidique.

Le processeur du système peut être adapté pour exécuter un programme informatique. Le système peut comprendre en outre une horloge interne ou un dispositif de minutage afin que le programme informatique puisse pour prendre en compte une variable liée au temps.

### Mode de fonctionnement :

Selon un aspect de l'invention qui peut utiliser un mode de réalisation précédemment divulgué, le système comprend un processeur et un capteur adaptés pour mesurer une pression associé à la pression intrapéritonéale (ou au moins pour surveiller l'évolution de la pression) au moins de façon temporaire durant une phase de stase. Cette surveillance permettra au système de retirer une quantité définie de dialysat selon certaines conditions de manière à garantir un certain confort au patient, limiter les effets indésirable ou potentiellement nocifs liés à une trop forte pression intrapéritonéale et/ou à améliorer sensiblement l'ultrafiltration.

Afin de limiter les retraits de dialysat suite à un mouvement du patient et/ou suite à une perturbation exogène, le système effectue la surveillance et/ou le retrait de dialysat durant une phase de stase en fonction d'au moins une condition suivante :
- une fenêtre de temps prédéfinie (c'est-à-dire un espace de temps dont le début, la fin et/ou la durée peut ou peuvent être prédéterminé(s)) et/ou
- une évolution de la pression et/ou
- la quantité d'ultrafiltrat estimée à un moment donné et/ou
- un ensemble de données traitées par un programme informatique.

Les données pris en compte par le système ou par le programme informatique du système peuvent être des variables quantitatives (mesure de pression, quantité d'ultrafiltrat estimée,...), catégorielles (fenêtre de temps ouverte ou fermée), discrètes (mesures aléatoires ou non) ou continues (mesure de pression continue).

### Exemple de « fenêtres de temps prédéfinies » :

La figure 3 représente différentes « fenêtres de temps prédéfinies ». T1 représente une phase de stase entière, le début de cette phase commence à l'extrémité gauche de la flèche et la fin de cette phase se termine par la pointe de la flèche. T2, T3, T4 et T4' représentent des « moments définis » de cette phase de stase durant lesquels le système va pouvoir effectuer un retrait de dialysat de la cavité péritonéale du patient. Si durant ces moments définis, les données relevées et/ou calculées par le processeur dépassent un seuil déterminé, le système pourra effectuer (de manière automatique par exemple) le retrait d'une quantité donnée de dialysat durant la phase de stase. Pour effectuer le retrait du dialysat, la pompe du système peut être utilisée.

Une fenêtre de temps peut être définie comme un laps de temps qui peut être prédéterminer, fixe ou variable selon certaine(s) condition(s). Une fenêtre de temps peut être caractérisée par une ouverture et/ou une fermeture. C'est-à-dire, qu'une fenêtre de temps peut s'ouvrir à un moment tₒ prédéfini et/ou se fermer à un moment t_{c} prédéfini. Préférentiellement, le système est adapté pour ouvrir au moins une fenêtre de temps à un moment tₒ₁ (tₒ₂, tₒ₃,... s'il y a plusieurs fenêtres de temps) d'une phase de stase.

Durant ce laps de temps défini, mais pas exclusivement, le système peut évaluer un ensemble de données par exemple la moyenne de pression pour un volume de remplissage donné, ce qui permet de s'affranchir des variations instantanées et plus particulièrement des variations instantanées qui se sont produites en dehors de ce laps de temps. Durant ce laps de temps défini, le système peut initier le retrait d'une fraction volumique de dialysat.

Ainsi, le retrait d'un certain volume de dialysat pourra être automatiquement commandé en fonction du volume de liquide (dialysat injecté durant la phase de remplissage plus ultrafiltrat produit durant la phase de stase) dans le péritoine du patient. Cela permet en outre de ne pas limiter l'ultrafiltration lorsque le volume de dialysat dans le péritoine est trop important.

Naturellement, au cours d'un même cycle, plusieurs retraits peuvent être effectués à différent moments en fonction de la pression intrapéritonéale mesurée.

T2 est une fenêtre de temps qui commence à un moment tₒ₂. Ainsi, la fenêtre de temps T2 s'ouvrira uniquement après un certain laps de temps suite au démarrage de la phase de stase. Selon la figure, T2 se termine à la fin de la phase de stase. Par exemple, T2 peut commencer 30 min après le début de la phase de stase. Si la phase de stase dure 120 min alors la durée de T2 sera de 90 min. Ce n'est que pendant ces 90 minutes que le système pourra, si nécessaire, effectuer un retrait de dialysat. Autrement dit, le patient pourra bouger pendant les 30 minutes qui précèdent cette fenêtre de temps de 90 minutes sans que le système ne déclenche intempestivement une opération de retrait du dialysat qui risquerait de rendre le traitement moins efficace.

T3 est une fenêtre de temps qui (comme T2) débute après qu'un certain laps de temps se soit écoulé suite au démarrage de la phase de stase. Toutefois T3 est une fenêtre de temps plus courte que T2 car T3 se termine avant la fin de la phase de stase. Ainsi, le système ne peut effectuer de retrait de dialysat après que cette fenêtre de temps se soit refermée, jusqu'à la fin de la phase de stase.

Dans le troisième exemple, la période durant laquelle le système peut procéder à des ajustements se compose de deux fenêtres de temps T4 et T4'. Leur durée est différente et leur moment d'activité est également différent et réparti sur une même phase de stase.

La durée d'une fenêtre de temps est strictement inférieure à la durée de la phase de stase qui comprend cette fenêtre de temps. Autrement-dit, la durée de la fenêtre de temps est égale à 1/x de la durée de la phase de stase, où x est strictement supérieur à 1. Par ailleurs, il va de même pour la durée totale de toutes les fenêtres de temps d'une même phase de stase.

Si le traitement comprend plusieurs phases de stase, le système peut ouvrir plusieurs fenêtres de temps à des moments identiques ou différents de chaque phase de stase.

Dans un mode de réalisation, la fenêtre de temps peut être fixe ou variable pour une phase de stase. Par exemple, si la fenêtre de temps est initialement de 90 minutes, mais que durant ces 90 minutes le système effectue un ou plusieurs retraits de dialysat, alors le système peut décider de réduire la fenêtre de temps à 75 minutes partant du principe que durant le temps restant la probabilité que la pression intrapéritonéale n'atteigne une nouvelle fois le seuil causé par l'ultrafiltration est trop faible. Ainsi, le patient pourra bouger, respirer sans déclenchement intempestif après la fin de la fenêtre de temps raccourci.

Le système peut avertir visuellement ou de façon sonore le patient lorsque ces moments définis sont actifs ainsi, le patient pourra choisir d'effectuer certain mouvement en dehors de ces moments définis. Par exemple, le système peut comprendre et afficher un écran de recommandation « Ne pas bouger pendant les mesures » ou « mesure en cours » ou « évaluation de la pression intrapéritonéale »... De cette manière le patient est informé que le système évalue l'opportunité d'effectuer ou non un retrait partiel du dialysat.

Selon un mode de fonctionnement compatible avec les précédents, le patient peut volontairement ouvrir une fenêtre de temps autrement dit le patient peut définir une fenêtre de temps par exemple s'il commence à avoir un certain inconfort, toutefois préférentiellement c'est le système qui pourra décider en fonction des données du capteur et/ou d'un ensemble de données (qui peut être pression) s'il initie ou non le retrait d'un certain volume de dialysat.

Inversement, le patient peut fermer temporairement une fenêtre de temps qui serait ouverte par le système, de manière à ce que le patient puisse bouger librement durant cette fermeture temporaire (désactivation temporaire). Le système peut rouvrir la fenêtre de temps après une durée déterminée (par le patient ou par le système), la durée de la fermeture temporaire peut modifier le moment T_{c} de la fermeture définitive de la fenêtre de temps modifiée. Afin de faciliter ce fonctionnement, le système peut comprendre une commande d'actionnement sur le cycler (par exemple bouton ou écran tactile) ou sur une télécommande distincte du cycler mais couplé avec ce dernier (avec ou sans fil).

Selon un mode de réalisation possible, le système peut également surveiller l'évolution de la pression en dehors de ces fenêtres de temps mais ne peut effectuer le retrait de dialysat uniquement pendant les fenêtres de temps. Autrement dit, le processeur prend en compte ou surveille un ensemble de données par exemple au moins deux mesures de pression, l'évolution de la pression, la moyenne de pression et/ou le profil de pression durant tout ou partie d'une phase de stase (ou plusieurs phases de stase) et actionne la pompe (ou autorise le retrait partiel du dialysat) uniquement lors de fenêtres de temps ouvertes.

### Surveillance de la pression :

Comme expliqué précédemment, les systèmes de l'état de l'art déclenchent le retrait d'une fraction du dialysat durant une phase de stase dès que une seule et unique valeur dépasse un seuil, par exemple dès que la pression intrapéritonéale dépasse un seuil. Or, la prise en compte d'une seule et unique valeur n'est pas souhaitable car elle engendre des déclenchements intempestifs du retrait de dialysat durant la phase de stases.

Etant donné que la pression intrapéritonéale varie en fonction de nombreux phénomènes (exogène et/ou endogène), il peut être nécessaire de prendre en compte non pas une valeur de pression mais un ensemble de données de manière à de filtrer les variations physiologiques ou liées à des mouvements du patient ou d'autres phénomènes. Idéalement seules les mesures stables, liées à un moment où le patient est immobile, devraient être prises en compte. Autrement dit, il faut écarter, ou du moins éviter de prendre en compte les variations instantanées et/ou parasitaires de la pression.

Afin que le système de l'invention n'effectue pas de retrait de dialysat de façon intempestive, le système surveille ou prend en compte un ensemble de données. Cet ensemble de données permet au système de ne pas déclencher, de façon inopportune, le retrait de dialysat durant la phase de stase. En effet, le système ordonnera le retrait que si cela est nécessaire. Pour cela, le système peut utiliser un processeur qui prend en compte plusieurs données. Par exemple, au moins deux valeurs de mesure du capteur (par exemple celles du capteur 309 de la figure 2) peuvent être analysées par le processeur.

Ainsi, le système peut prendre en compte plusieurs valeurs liées à la pression intrapéritonéale. Ces valeurs peuvent être les mesures de capteur de pression par exemple des mesures continues, aléatoires, des pics de pression (positif et/ou négatif ou minimum et/ou maximum). Concernant les pics de pression, le système peut définir des moments où chaque moment peut comprendre deux pics opposés ou non. Pour discerner ces pics, le système peut utiliser les dérivées premières et/ou secondes de la courbe de pression mesurée. Ou plus simplement, durant un lapse de temps définit, le système peut définir une valeur maximal et/ou une valeur minimal.

Selon un mode de fonctionnement, le modèle mathématique peut prendre en compte une mesure de pression effectué à un moment prédéfini (par exemple : en fin de phase de remplissage précédent la phase de stase ou au début de la phase de stase). Cette mesure peut être utilisée comme valeur de référence servant à calculer un seuil de pression ou un gradient de pression permettant d'initier le retrait partiel du dialysat. Ainsi, dans ce cas, le système utilise un modèle mathématique utilisant au moins deux valeurs, une valeur de référence et une valeur de mesure à un temps t au cours de la phase de stase qui est comparé (par exemple) à la valeur de référence.

Le système peut également utiliser un modèle mathématique prenant en compte ces valeurs. Par exemple, le modèle mathématique peut calculer une moyenne. Cette moyenne peut être une moyenne des données de pression durant un cycle par exemple durant un cycle de respiration (inspiration + expiration) du patient, ou tout autre cycle de temps par exemple un cycle peut être compris entre deux pics de pression maximum et/ou minimum ou il peut s'agir également d'une moyenne mobile.

Par exemple, la figure 4 expose 3 séries de données, la première série de données est représentée par la courbe en pointillé. Il s'agit ici des données brutes de mesure du capteur. La deuxième série de données, représentée par une courbe en tiret, montre, par exemple, une moyenne mobile des données du capteur sur une période prédéfinie. La troisième est un seuil. Les dispositifs de l'état de l'art déclencheront le retrait d'une partie du dialysat dès que la courbe en pointillé atteindra ou dépassera le seuil, soit de nombreuses fois durant la phase de stase. Aussi, ces dispositifs risquent de retirer trop de dialysat durant une unique phase de stase, ce qui aura un impact substantiel sur l'efficacité du traitement. Le dispositif de l'invention ne déclenchera le retrait, par exemple, que si la courbe en tiret atteint ou dépasse le seuil. Dans cet exemple, le dispositif n'effectuera qu'un seul retrait de manière à optimiser l'ultrafiltration, la sécurité du patient ou son confort.

Selon un autre mode de fonctionnement (qui peut être utilisé avec le présent), un autre moyen pour initier le déclenchement du retrait partiel peut être de surveiller la tendance de l'évolution de la pression, aussi, le modèle mathématique peut être adapté pour définir une tendance de l'évolution de la pression de manière à commander automatiquement pendant la phase de stase un retrait partielle du dialysat. Par exemple, les points P1 et P2 (de la figure 4) ne représentent pas une tendance, il ne s'agit vraisemblablement que de pics de pression peut être dus au(x) mouvement(s) du patient. Par-contre, après ces deux pics de pression, la courbe des mesures de pression suit une tendance haussière entre les deux traits parallèles ce qui est caractéristique d'une augmentation moyenne de la pression intrapéritonéale causée par l'ultrafiltration. Grâce à cette méthode le système peut déterminer s'il doit ou non effectuer le retrait de dialysat.

La surveillance de la tendance peut permettre de discriminer des variations de pression qui ne seraient pas dues à l'ultrafiltration. Ainsi, le modèle mathématique pourrait prendre en compte un ensemble de valeurs de manière à calculer une tendance de l'évolution de la pression, par exemple en utilisant les modèles statistiques connu par l'homme du métier tel que les modèles de régression (linéaire, linéaire multiple, polynomiale, linéaire généralisé non paramétrique, multiple postulés et non postulés, circulaire, elliptique ou locale,...), une méthode de moindre carré, l'écart-type, la variance, l'espérance mathématique,... Grâce à ce modèle mathématique, le système pourrait permettre également de prédire à quel moment il serait opportun d'effectuer un retrait partiel de dialysat. Autrement dit, ce modèle mathématique pourrait prédire un moment pour effectuer un retrait partiel de manière à définir une fenêtre de temps (par exemple tₒ, t_{c} ou durée) tel que discuté en amont.

Le système peut également prendre en compte d'autre valeur qui serait lié directement ou non à la pression intrapéritonéale, telle que la température de l'environnement ou du patient (dans ce cas, le système peut comprendre un capteur de température), l'activité du patient (par exemple en fonction de l'heure, sommeil, ...), son état de santé ou son profil (qui pourrait être renseigner avant le traitement grâce à l'interface utilisateur du système (maladie, âge, sexe, volume maximale ou acceptable pour le péritoine du patient,...)),...

### Volume dialysat ou d'ultrafiltrat :

Durant le retrait partiel, les systèmes de l'état de l'art surveillent la pression afin d'arrêter le retrait dès que la mesure de pression atteint un seuil prédéfini. Or, tout comme pour le déclenchement du retrait, la pression n'est pas une donnée suffisamment fiable car elle varie trop en fonction de divers phénomènes. Ainsi, pour palier à ces défauts, le système de l'invention peut prendre en compte un volume d'ultratfiltrat produit à un moment donné ou peut déterminer ou estimer un volume dialysat à retirer ou peut retirer un volume prédéfini de dialysat durant la phase de stase.

Ainsi, le système de l'invention peut retirer une quantité prédéfinie de dialysat. Cette quantité peut être un certain volume, par exemple 400 ml, ou un pourcentage de dialysat injecté, par exemple 20% du dialysat injecté lors d'une précédente phase de Fill, ce pourcentage pouvant être compris entre 0 et 100%, préférentiellement entre 5 et 50%. Cette quantité peut être déterminée par le médecin ou estimer par le système en prenant en compte la tendance de la courbe de pression ou de la valeur de référence cité ci-avant ou en fonction des données patients (profil patient, état de santé, caractéristique du péritoine, du dialysat (niveau de glucose), efficacité du traitement, compliance au traitement, ...).

Le système peut ainsi être adapté pour calculer ou estimer le volume (ou quantité) d'ultrafiltrat présent (ou produit) dans la cavité péritonéale à un moment donné par exemple juste avant ou lors du retrait. Cette quantité peut être une estimation basée sur un modèle mathématique. Ce modèle mathématique peut être utilisé pour calculer un volume d'ultrafiltrat au cours du temps ou une tendance. Ce modèle mathématique peut prendre un compte des données empiriques, une table de données, ou toutes autres données citées ci-avant.

Cette fraction volumique à retirer durant la phase de stase peut prendre en compte cette estimation afin de retirer :
- un volume de dialysat équivalent (substantiellement) à la quantité estimé d'ultrafiltrat à un moment t, ou
- un plus grand volume de dialysat que l'ultrafiltrat, ou
- un plus petit volume de dialysat que l'ultrafiltrat.

Le modèle mathématique peut également prendre en compte le temps restant avant la fin de la phase de stase.

En outre, cette donnée peut également être prise en compte par le système (par exemple un modèle mathématique) pour l'initiation du retrait. Ainsi le système pourrait initier le retrait dès lors que le volume estimé d'ultrafiltrat produit dépasse un certain seuil ou dès lors que le volume estimé de liquide présent dans le péritoine du patient dépasse un seuil prédéterminé.

Ainsi, l'invention porte sur un système pour traitement de dialyse péritonéale à un patient, le système comprenant :
∘ Un circuit de dialysat adapté pour être fluidiquement connecté à la cavité péritonéale du patient durant le traitement de dialyse,
∘ Un dispositif de pompage adapté pour faire s'écouler du dialysat à travers le circuit de dialysat vers ou depuis de la cavité péritonéale du patient,
∘ Un capteur adapté pour mesurer la pression du dialysat, le capteur étant agencé de manière à mesurer une pression associée à la pression intrapéritonéale pendant une phase de stase,
∘ Un processeur couplé à la pompe et au capteur
∘ Un programme informatique exécutable par le processeur et prenant en compte les valeurs de mesure du capteur,
le processeur étant configuré pour exécuter les instructions suivantes:
- recevoir les valeurs de mesure du capteur,
- Exécuter le programme informatique en utilisant un modèle mathématique prenant en compte plusieurs valeurs de mesures de pression durant une phase de stase, et
- Initier le retrait d'une fraction volumique de dialysat de la cavité péritonéale pendant une phase de stase en fonction du résultat du programme informatique montrant que les données relevées et/ou calculées par le processeur dépassent un seuil déterminé et uniquement pendant une fenêtre de temps définie de la phase de stase dont la durée est inférieure à la durée de la phase de stase.

Des modes de réalisation préférés sont définis par les revendications dépendantes.

## Revendications

1. Système pour traitement de dialyse péritonéale à un patient, le système comprenant :
un circuit de dialysat adapté pour être fluidiquement connecté à la cavité péritonéale du patient durant le traitement de dialyse,
un dispositif de pompage adapté pour faire s'écouler du dialysat à travers le circuit de dialysat vers ou depuis la cavité péritonéale du patient,
un capteur adapté pour mesurer la pression du dialysat, le capteur étant agencé de manière à mesurer une pression associée à la pression intrapéritonéale pendant une phase de stase,
un processeur couplé à la pompe et au capteur, et
un programme informatique exécutable par le processeur et prenant en compte les valeurs de mesure du capteur,
le processeur étant configuré pour exécuter les instructions suivantes :
• recevoir les valeurs de mesure du capteur,
• exécuter le programme informatique en utilisant un modèle mathématique prenant en compte plusieurs valeurs de mesures de pression durant une phase de stase, et
• initier le retrait d'une fraction volumique de dialysat de la cavité péritonéale pendant une phase de stase en fonction du résultat du programme informatique montrant que les données relevées et/ou calculées par le processeur dépassent un seuil déterminé et uniquement pendant une fenêtre de temps définie de la phase de stase dont la durée est inférieure à la durée de la phase de stase.

2. Le système selon la revendication 1, dans lequel le modèle mathématique prend uniquement en compte les valeurs de mesure pendant un temps déterminé.

3. Le système selon l'une des précédentes revendications, dans lequel le modèle mathématique prend uniquement en compte la ou les valeurs de mesure extrêmes pendant un ou plusieurs moments définis durant une même phase de stase.

4. Le système selon l'une des précédentes revendications, dans lequel le modèle mathématique calcule une moyenne des valeurs de mesure prises en compte.

5. Le système selon la revendication 4, dans lequel la moyenne calculée est une moyenne mobile.

6. Le système selon l'une des précédentes revendications, dans lequel le système est autorisé à initier le retrait d'une fraction volumique de dialysat de la cavité péritonéale durant la fenêtre de temps.

7. Le système selon la revendication 6, dans lequel la fenêtre de temps est **caractérisée par** une ouverture prédéterminée et/ou une fermeture prédéterminée et/ou une durée prédéterminée.

8. Le système selon l'une des précédentes revendications, dans lequel le programme informatique ne prend pas en compte les variations de valeurs qui seraient dues à un mouvement du patient, à la respiration du patient ou aux battements du cœur du patient.

9. Le système selon l'une des précédentes revendications, dans lequel le programme informatique prend en outre en compte une estimation du volume d'ultrafiltrat au moment de l'exécution de programme.

10. Le système selon l'une des précédentes revendications, dans lequel le processeur est configuré pour exécuter en outre les instructions suivantes :
• mesurer la pression à un moment prédéfini, et
• enregistrer cette valeur dans une mémoire du système, cette valeur étant considérée comme une valeur de référence.

11. Le système selon la revendication 10, dans lequel le moment prédéfini est en fin de phase de remplissage ou début de phase de stase.

12. Le système selon la revendication 10, dans lequel le processeur est configuré pour exécuter en outre l'instruction suivante :
• comparer une valeur de référence à d'autres valeurs liées à la pression du dialysat durant la phase de stase.

13. Le système selon l'une des précédentes revendications 10 à 12, dans lequel le processeur est configuré pour exécuter en outre les instructions suivantes :
• calculer un seuil de pression en fonction d'une valeur de référence.

14. Le système selon l'une des précédentes revendications 10 à 13, dans lequel le processeur est configuré pour exécuter en outre les instructions suivantes :
• calculer un gradient de pression en fonction d'une valeur de référence.

15. Le système selon la revendication 13 ou 14, dans lequel le processeur est configuré pour exécuter en outre l'instruction suivante :
• initier le retrait dès qu'une valeur liée à la pression du dialysat atteint le seuil de pression calculé ou le gradient de pression calculé.

## Patentansprüche

1. System zur Peritonealdialysebehandlung eines Patienten, wobei das System umfasst:
einen Dialysatkreislauf, der so ausgelegt ist, dass er während der Dialysebehandlung mit der Bauchhöhle des Patienten fluidverbunden ist,
eine Pumpvorrichtung, die so ausgelegt ist, dass sie Dialysat durch den Dialysatkreislauf hindurch in Richtung der Bauchhöhle des Patienten oder von der Bauchhöhle des Patienten weg strömen lässt,
einen Sensor, der so ausgelegt ist, dass er den Dialysatdruck misst, wobei der Sensor so angeordnet ist, dass er einen Druck misst, der mit dem Druck im Bauchraum während einer Verweilphase verknüpft ist,
einen Prozessor, der mit der Pumpe und dem Sensor gekoppelt ist, und
ein Computerprogramm, das von dem Prozessor ausführbar ist und die Messwerte des Sensors berücksichtigt,
wobei der Prozessor so eingerichtet ist, dass er folgende Befehle ausführt:
▪ Empfangen der Messwerte des Sensors,
▪ Ausführen des Computerprogramms unter Verwendung eines mathematischen Modells, das mehrere Druckmesswerte während einer Verweilphase berücksichtigt, und
▪ Einleiten des Entnehmens eines Volumenanteils des Dialysats der Bauchhöhle während einer Verweilphase in Abhängigkeit vom Ergebnis des Computerprogramms, das anzeigt, dass die von dem Prozessor erfassten und/oder berechneten Daten einen vorgegebenen Schwellenwert übersteigen, und ausschließlich während eines festgelegten Zeitfensters der Verweilphase, dessen Dauer kürzer ist als die Dauer der Verweilphase.

2. System nach Anspruch 1, wobei das mathematische Modell ausschließlich die Messwerte während eines bestimmten Zeitabschnitts berücksichtigt.

3. System nach einem der vorhergehenden Ansprüche, wobei das mathematische Modell nur den extremen Messwert oder nur die extremen Messwerte während eines festgelegten Zeitpunkts oder mehrerer festgelegter Zeitpunkte während ein und derselben Verweilphase berücksichtigt.

4. System nach einem der vorhergehenden Ansprüche, wobei das mathematische Modell einen Mittelwert der berücksichtigten Messwerte berechnet.

5. System nach Anspruch 4, wobei der berechnete Mittelwert ein gleitender Mittelwert ist.

6. System nach einem der vorhergehenden Ansprüche, wobei das System dazu berechtigt ist, während des Zeitfensters das Entnehmen eines Volumenanteils des Dialysats der Bauchhöhle einzuleiten.

7. System nach Anspruch 6, wobei das Zeitfenster durch eine vorgegebene Öffnung und/oder eine vorgegebene Schließung und/oder eine vorgegebene Dauer gekennzeichnet ist.

8. System nach einem der vorhergehenden Ansprüche, wobei das Computerprogramm keine Werteveränderungen berücksichtigt, die auf eine Bewegung des Patienten, die Atmung des Patienten oder Herzschläge des Patienten zurückzuführen wären.

9. System nach einem der vorhergehenden Ansprüche, wobei das Computerprogramm ferner eine Schätzung des Ultrafiltratvolumens zum Zeitpunkt der Ausführung des Programms berücksichtigt.

10. System nach einem der vorhergehenden Ansprüche, wobei der Prozessor so eingerichtet ist, dass er ferner folgende Befehle ausführt:
▪ Messen des Drucks zu einem vorher festgelegten Zeitpunkt, und
▪ Speichern dieses Werts in einem Speicher des Systems, wobei dieser Wert als ein Bezugswert betrachtet wird.

11. System nach Anspruch 10, wobei der vorher festgelegte Zeitpunkt am Ende der Füllphase oder zu Beginn der Verweilphase liegt.

12. System nach Anspruch 10, wobei der Prozessor so eingerichtet ist, dass er ferner folgenden Befehl ausführt:
▪ Vergleichen eines Bezugswerts mit weiteren Werten, die mit dem Dialysatdruck verbunden sind, während der Verweilphase.

13. System nach einem der vorhergehenden Ansprüche 10 bis 12, wobei der Prozessor so eingerichtet ist, dass er ferner folgende Befehle ausführt:
▪ Berechnen eines Druckschwellenwerts in Abhängigkeit von einem Bezugswert.

14. System nach einem der vorhergehenden Ansprüche 10 bis 13, wobei der Prozessor so eingerichtet ist, dass er ferner folgende Befehle ausführt:
▪ Berechnen eines Druckgefälles in Abhängigkeit von einem Bezugswert.

15. System nach Anspruch 13 oder 14, wobei der Prozessor so eingerichtet ist, dass er ferner folgenden Befehl ausführt:
▪ Einleiten des Entnehmens, sobald ein mit dem Dialysatdruck verbundener Wert den berechneten Druckschwellenwert oder das berechnete Druckgefälle erreicht.

## Claims

1. System for peritoneal dialysis treatment to a patient, the system comprising:
a dialysate circuit adapted to be fluidically connected to the peritoneal cavity of the patient during the dialysis treatment,
a pumping device adapted for causing dialysate to flow through the dialysate circuit to or from the peritoneal cavity of the patient,
a sensor adapted for measuring the pressure of the dialysate, the sensor being configured in such a way as to measure a pressure associated with the intraperitoneal pressure during a stasis phase,
a processor coupled to the pump and to the sensor,
a computer program that can be executed by the processor and takes account of the measurement values from the sensor, the processor being configured to execute the following instructions:
• receiving the measurement values from the sensor,
• executing the computer program using a mathematical model that takes account of several pressure measurement values during a stasis phase, and
• initiating the withdrawal of a dialysate volume fraction from the peritoneal cavity during a stasis phase, according to the result of the computer program, showing that the data collected and/or calculated by the processor exceed a determined threshold and only during a defined time window of the stasis phase whose duration is less than the duration of the stasis phase.

2. System according to Claim 1, in which the mathematical model only takes account of the measurement values during a determined time.

3. System according to either of the preceding claims, in which the mathematical model only takes account of the one or more extreme measurement values during one or more defined times during one and the same stasis phase.

4. System according to one of the preceding claims, in which the mathematical model calculates an average of the measurement values taken into account.

5. System according to Claim 4, in which the calculated average is a moving average.

6. System according to one of the preceding claims, in which the system is authorized to initiate the withdrawal of a dialysate volume fraction from the peritoneal cavity during the time window.

7. System according to Claim 6, in which the time window is **characterized by** a predetermined opening and/or a predetermined closing and/or a predetermined duration.

8. System according to one of the preceding claims, in which the computer program does not take account of the variations in values that would be due to a movement of the patient, to the breathing of the patient or to the beating of the patient's heart.

9. System according to one of the preceding claims, in which the computer program additionally takes account of an estimate of the volume of ultrafiltrate at the moment of program execution.

10. System according to one of the preceding claims, in which the processor is configured to execute the additional following instructions:
• measuring the pressure at a predefined moment, and
• registering this value in a memory of the system, this value being considered as a reference value.

11. System according to Claim 10, in which the predefined moment is at the end of the filling phase or the start of the stasis phase.

12. The method according to Claim 10, in which the processor is configured to execute the additional following instruction:
• comparing a reference value to other values linked to the pressure of the dialysate during the stasis phase.

13. System according to one of the preceding Claims 10 to 12, in which the processor is configured to execute the additional following instructions:
• calculating a pressure threshold as a function of a reference value.

14. System according to one of the preceding Claims 10 to 13, in which the processor is configured to execute the additional following instructions:
• calculating a pressure gradient as a function of a reference value.

15. System according to Claim 13 or 14, in which the processor is configured to execute the additional following instruction:
• initiating the withdrawal as soon as a value linked to the pressure of the dialysate reaches the calculated pressure threshold or the calculated pressure gradient.
